# EUROPEAN PATENT APPLICATION

(11) **EP 3 058 985 A1**
(43) Date of publication of application: **24.08.2016**
(21) Application number: 14854218.6
(22) Date of filing: 20.10.2014
(51) Int. Cl.: A61N 2/00, A61N 1/36

(54) **ELECTRIC OR MAGNETIC STIMULATION DEVICE FOR TREATMENT OF CIRCULATORY DISEASE**

(30) Priority: 18.10.2013 US 201361892609 P
(71) Applicant: Kyushu University National University Corporation, Fukuoka-shi Fukuoka 812-8581 (JP)
(72) Inventor: SUNAGAWA, Kenji, Fukuoka-shi, Fukuoka 812-8581 (JP); IDE, Tomomi, Fukuoka-shi, Fukuoka 812-8581 (JP); SAKAMOTO, Kazuo, Fukuoka-shi, Fukuoka 812-8581 (JP)
(74) Representative: Dunleavy, Kevin James
(86) International application number: PCT/JP2014/077853
(87) International publication number: WO 2015/056810

(57) **Abstract**

The purpose of the present invention is, when treating circulatory disease such as acute myocardial infarction, to correct a reduction in myocardial contractility and thereby prevent arrhythmia and the like, as well as to reduce infarct size. Provided is a neurostimulation device having a stimulus application part configured so as to apply a stimulus to a cervical or thoracic vagus nerve portion in a human or an animal, and a stimulus regulation part configured so as to regulate the quantity of stimulus applied from the stimulus application part to the vagus nerve portion. The stimulus regulation part establishes a quantity of stimulus that is less than a value at which adverse effects would be produced, determines the heart rate and R wave interval of the human or animal, and controls the quantity of stimulus on the basis of these determinations.

## Description

### FIELD OF THE INVENTION

The present invention relates to an electric or magnetic stimulation device for the treatment of circulatory disease in a human or an animal as well as to a method for treating the circulatory disease in the human or animal.

More specifically, the electric stimulation device for the treatment of circulatory disease relating to the present invention comprises an electrode and an electric stimulus application means for applying an electric stimulus to a cervical vagus nerve of an animal and is capable of treating circulatory disease by applying an electric stimulus to the vagus nerve. Moreover, in the method for treating circulatory disease relating to the present invention, the electrode is disposed in direct contact with a cervical vagus nerve of an animal, and circulatory disease is treated by applying an electric stimulus signal to the vagus nerve through this electrode.

Moreover, by applying a stimulus to a cervical vagus nerve or the like with magnetic pulses extracorporeally using a magnetic pulse generator, circulatory disease is treated in a manner similar to the abovementioned electric stimulation.

### BACKGROUND OF THE INVENTION

Myocardial infarction is one of ischemic heart disorders and refers to a state in which the flow rate of coronary blood, nutrients for the heart, declines and thereby cardiac muscles are made ischemic and then become necrotic. It usually refers to acute myocardial infarction or AMI, which occurs acutely. As a therapeutic method, it is a general rule to have absolute bed rest in the acute phase. In the acute phase after the onset, it is highly likely that fatal arrhythmia tends to occur, which leads to death. Moreover, as a period of ischemia prolongs, the death of cardiac muscles progresses, resulting in an irreversible decline in the cardiac function. If there is any possibility of the onset, it is necessary to keep a close eye on the patient and call for an ambulance, and if the patient loses consciousness and pulse is impalpable, it is necessary to perform cardiac massage without hesitation. In case functional cardiac arrest occurs, the rehabilitation rate becomes almost nil if no treatment is performed for 3-5 minutes or more. It is necessary to perform life support (cardiac massage or the like) immediately without waiting for the arrival of emergency crew.

Myocardial infarction is caused by insufficient supply of oxygen to cardiac muscles relatively and absolutely, the patient needs to be at rest and oxygen inhalation should be performed as a therapeutic method. Moreover, morphine may be administered for the purposes of pain relief and a reduction in oxygen consumption by the body. In the acute phase, it is the most important to prevent lesions of myocardial infarction from spreading. Generally, "aspirin administration," "oxygen inhalation," "morphine administration," "nitric acid medicine" and the like are mainly performed, and these treatments are referred to as "MONA" by taking the initial letters of morphine, oxygen, nitrate and aspirin and are well-known as the first aid of myocardial infarction.

In the case of myocardial infarction within 6 hours of the onset, it is possible to reduce necrotic areas of cardiac muscles by actively performing reperfusion therapy on an occluded coronary artery. Besides, in cases within 24 hours of the onset, it is believed to be meaningful to perform reperfusion therapy. The treatment is largely classified into percutaneous coronary intervention (PCI) and percutaneous transluminal coronary recanalization (PTCR), and treatment strategies vary depending on country, insurance and doctors' judgment. PCI is a therapeutic method in which a thin tube called a catheter is inserted into a blood vessel from a hole having a several millimeters in diameter opened mainly on the skin. In Japan, many facilities are capable of performing emergency PCI and, therefore, PCI is frequently performed in the acute phase. However, since this is an examination and treatment method via an artery, complications are not rare, and therefore this method requires skilled procedure by specialists. In isolated island, the survival rate is lower than medically sparsely populated areas for the reason of difficulty in transportation. In other words, in the case of acute myocardial infarction, it is significant to perform PCI as soon as possible. Hospitals capable of performing the therapy immediately after emergency hospitalization is rare even in the United States of America, an advanced country for the treatment of cardiac disorders. There are some facilities that perform emergency coronary artery bypass grafting (CABG) when there are three or more stenotic segments. When PCI is compared with CABG, some people believe that CABG is more advantageous even when there is only one branch, because it is believed that restenosis occurs in 25-30% of lesions treated by PCI. However, it is expected that PCI results will be improved because health-care insurance became applicable to drug-eluting stent or DES in 2004. If the intervention therapy including PCI is successful in the acute phase, prognosis can comparatively be kept well. The intervention therapy came into the limelight recently as a therapeutic method that only puts a very small burden on patients. Since a scar is small, recovery is quick after surgery and patients can be discharged from the hospital after only a short hospital stay (3-5 days), so that the QOL (quality of life) of patients can significantly be improved, and it is also said that this is a therapeutic method that can reduce economic burdens of patients and also contribute to a policy of reducing medical costs sponsored by the government. However, in reperfusion therapy such as intervention, complications frequently occur including arrhythmia, extrasystole, ventricular fibrillation, atrioventricular block and cardiac failure.

Although it is indispensable to maintain life, quick recovery of circulation also exposes the body to a danger. Reperfusion not only increases topical damage but produces inflammation reaction, which produces systemic insult as well. Acute onsets such as myocardial infarction, stroke, cardiac failure, arrhythmia and cardiac arrest could produce ischemia-reperfusion injury (IRI). However, many of scheduled surgical treatments such as organ implantation and the treatment of aneurysm require an ischemic period during the treatment, and thereby IRI could be produced. It used be believed that the presence of inflammatory cells in the ischemic tissue shows pathophysiological reaction to injury. However, studies and experiments have shown that although the presence of inflammatory cells is important for healing, an inflow of inflammatory cells and particularly macrophages, which are phagocytic cells, into the tissue produces tissue injury that goes beyond the tissue injury caused by ischemia alone. Such injury could affect a wide variety of tissues such as the heart, brain, liver, spleen, intestines, lungs and pancreas.

Various methods have been reported for preventing reperfusion injury including induced hypothermia, controlled reperfusion and ischemic preconditioning. The induced hypothermia means the introduction of moderate hypothermia (28-32°C) to patients. It is believed that mild hypothermia can suppress a great deal of reperfusion-related chemical reactions. Despite these potential advantages, hypothermia brings about various adverse effects such as arrhythmia, infection and blood clotting. The controlled reperfusion means the control of the initial phase of reperfusion by performing reperfusion on the tissue under low pressure using blood that has been modified so as to have hyperosmosis, alkalosis and concentrated substrates. The ischemic preconditioning means the intentional onset of ischemia for a short period so as to achieve protective effects during a longer period of ischemia by slowing down the metabolism of cells. Although they could be useful in surgical setting (e.g., before and after a scheduled cardiac operation), these treatments are not usually appropriate because they are used mainly in a control-required fixed situation.

It was recently reported that, as a therapeutic method for chronic cardiac failure, it would be effective to apply an electric stimulus to a vagus nerve. In other words, since the heart rate declines when an electric stimulus is applied to a vagus nerve, this method is to prevent or ameliorate the oxygen deprivation of cardiac muscles by lowering the quantity of oxygen consumption of cardiac muscles as a result of the declined heart rate. In consequence, the occurrence of ischemia in cardiac muscles and fatal arrhythmia associated therewith can be prevented, and therefore it is believed that this method is effective in treating and preventing cardiac failure. The technology of a vagus nerve stimulation system for applying an electric stimulus to a vagus nerve and particularly the technology of a vagus nerve stimulation system capable of stimulating a vagus nerve subcutaneously or indirectly through the body surface has been disclosed in the following patent literature.

Japanese Patent Application Kohyo Publication No. 2005-500863 and Japanese Patent Application Kokai Publication No. 2009-233024

Moreover, in the abovementioned vagus nerve stimulation systems, the intensity of stimulation is determined based on a decline in the heart rate. However, vagal nerve stimulation is accompanied by adverse effects though it depends on where the stimulation is applied, and therefore, in order to reduce adverse effects, a decline in the heart rate is suppressed as much as possible, or the intensity of stimulation is blindly set to a level at which the heart rate does not decline.

### SUMMARY OF THE INVENTION

In view of the abovementioned circumstances, the present invention was designed, and the purpose of the present invention is to provide a novel therapeutic method and a therapeutic device, which are capable of correcting a reduction in myocardial contractility and thereby preventing arrhythmia and the like, as well as reducing infarct, when treating circulatory disease such as acute myocardial infarction.

In order to solve the abovementioned problems, according to a first major viewpoint of the present invention, provided is a neurostimulation device configured so as to stimulate a cervical or thoracic vagus nerve portion in a human or an animal with a controlled quantity of stimulus by applying electric signals to an electrode or a magnetic coil attached to the cervical or thoracic portion in the human or animal, the neurostimulation device comprising: a biological sign value detection part for detecting biological sign values of the human or animal; a threshold storage part for storing thresholds of the biological sign values; a stimulus reduction quantity/reduction rate storage part for storing the reduction quantity/reduction rate of the quantity of stimulus; a first stimulus quantity determination part for determining a first quantity of stimulus obtained from a threshold stored in the threshold storage part, based on a biological sign value detected by the biological sign value detection part; a second stimulus quantity determination part for determining a second quantity of stimulus obtained by reducing the first quantity of stimulus by a reduction quantity/reduction rate stored in the stimulus reduction quantity/reduction rate storage part; and a control part for controlling the neurostimulation device in such a manner as to stimulate the cervical or thoracic vagus nerve portion in the human or animal by the second quantity of stimulus thus determined.

The present invention prevents complications such as a reduction in myocardial contractility and arrhythmia by performing vagal nerve stimulation in condition where there occurs less adverse effects and high therapeutic effects can be achieved using a novel heart rate stability index capable of confirming the effect of vagal nerve stimulation. Moreover, the present invention proposes a control system for regulating the intensity of stimulation at the time of stimulating a vagus nerve electrically or magnetically. In place of the heart rate, another biological sign value may be used including blood pressure value, respiratory rate and body temperature.

Moreover, in one embodiment of the present invention, the control part comprises a stimulus quantity regulation part that regulates the first quantity of stimulus based on the biological sign value while detecting the biological sign value.

In another embodiment of the present invention, the present device further comprises a reduction quantity/reduction rate input interface for the quantity of stimulus, so that a user can input a reduction quantity/reduction rate of the quantity of stimulus.

In another embodiment of the present invention, the reduction quantity/reduction rate of the quantity of stimulus is about 50%.

In another embodiment of the present invention, the device further comprises a biological sign value threshold input interface, so that a user can input a threshold of the biological sign value.

In another embodiment of the present invention, the threshold is about 10%.

In another embodiment of the present invention, the biological sign value to be detected by the biological sign value detection part is a heart rate. In this case, the biological sign value detection part detects an R wave interval of heartbeats in addition to the heart rate, and the control part calculates a fluctuation in the R wave interval, determines whether or not the fluctuation in the R wave interval is smaller than a predetermined fluctuation and, if it is not smaller, regulates the second quantity of stimulus until the R wave interval becomes smaller than the predetermined fluctuation.

Furthermore, in this case, the predetermined fluctuation is a fluctuation of the R wave interval at a time when no stimulation is applied.

In another embodiment of the present invention, the control part controls the neurostimulation device in such a manner that a stimulus can be applied intermittently or continuously based on the second quantity of stimulus. In this case, the control part applies a stimulus at a stimulation cycle in which the stimulus is applied continuously for about 10 seconds per minute and no stimulus is applied for the remaining 50 seconds or so.

In another embodiment of the present invention, the control part controls the neurostimulation device in such a manner that stimulation can be applied before reperfusion, after reperfusion or in combination with reperfusion.

In another embodiment of the present invention, the control part controls the neurostimulation device in such a manner as to apply a stimulus at a frequency of about 20Hz.

In another embodiment of the present invention, the stimulus includes electric pulses and the quantity of stimulus is a voltage.

In another embodiment of the present invention, the stimulus includes electric pulses and the quantity of stimulus is an electric current.

In another embodiment of the present invention, the stimulus includes magnetic pulses and the quantity of stimulus is a magnetic flux density. In this case, the control part regulates the magnetic flux density in such a manner that electric current generated in the biological tissue by the magnetic flux density is 15mA/cm² or less.

According to a second major viewpoint of the present invention, provided is a nerve stimulation method for stimulating a cervical or thoracic vagus nerve portion in a human or an animal, the method comprising: a biological sign value detection step of detecting a biological sign value of the human or animal; a threshold storage step of storing a threshold of biological sign values; a stimulus reduction quantity/reduction rate storage step of storing the reduction quantity/reduction rate of the quantity of stimulus; a first stimulus quantity determination step of determining a first quantity of stimulus obtained from a threshold stored in the threshold storage step, based on a biological sign value detected in the biological sign value detection step; a second stimulus quantity determination part for determining a second quantity of stimulus obtained by reducing the first quantity of stimulus by a reduction quantity/reduction rate stored in the stimulus reduction quantity/reduction rate storage part; and a control step of controlling the quantity of stimulus in such a manner as to stimulate the cervical or thoracic vagus nerve portion in the human or animal by the second quantity of stimulus thus determined.

Such a constitution can provide a nerve stimulation method that can be implemented by a neurostimulation device according to the abovementioned first viewpoint.

The other characteristics of the present invention in addition to those described above can readily be appreciated by those skilled in the art by referring to "detailed description of the invention" as well as drawings described below.

### BRIEF DESCRIPTION OF THE DRAWINGS

Fig. 1 is a schematic block diagram showing a neurostimulation device according to one embodiment of the present invention.
Fig. 2 is an experimental protocol according to one embodiment of the present invention.
Fig. 3 is graphs showing changes in the heart rate caused by vagal nerve stimulation. Stimulation voltage 100% is defined as a 0.5V lower value than a voltage value of vagal nerve stimulation that lowers the heart rate by 10%. Specific voltage values vary depending on individuals.
Fig. 4 is a graph produced by plotting and comparing values obtained by measuring changes in the heart rate every week for four weeks in four groups of vagal nerve stimulation with sham stimulation (SS) and voltage values 25 %, 50% and 100%.
Fig. 5 is a graph produced by comparing values of average blood pressure measured on Day 28 in four groups of vagal nerve stimulation with sham stimulation (SS) and voltage values 25 %, 50% and 100%.
Fig. 6 is graphs produced by comparing values of biventricular weight and lung weight measured on Day 28 in four groups of vagal nerve stimulation with sham stimulation (SS) and voltage values 25 %, 50% and 100%.
Fig. 7 is graphs produced by comparing values of left ventricular end-diastolic pressure and left ventricular dP/dt measured on Day 28 in four groups of vagal nerve stimulation with sham stimulation (SS) and voltage values 25 %, 50% and 100%.
Fig. 8 is graphs produced by comparing values of left ventricular ejection fraction and quantities of brain natriuretic peptide (BNP) measured on Day 28 in four groups of vagal nerve stimulation with sham stimulation (SS) and voltage values 25 %, 50% and 100%.
Fig. 9 is graphs produced by comparing values of biventricular weight and lung weight measured at Week 4 in four groups of vagal nerve stimulation with sham stimulation (SS) and frequencies 5Hz, 10Hz and 20Hz.
Fig. 10 is graphs produced by comparing values of left ventricular end-diastolic pressure and left ventricular dP/dt measured at Week 4 in four groups of vagal nerve stimulation with sham stimulation (SS) and frequencies 5Hz, 10Hz and 20Hz.
Fig. 11 is graphs produced by comparing values of left ventricular ejection fraction and quantities of brain natriuretic peptide (BNP) measured at Week 4 in four groups of vagal nerve stimulation with sham stimulation (SS) and frequencies 5Hz, 10Hz and 20Hz.
Fig. 12 is graphs produced by comparing values of biventricular weight and lung weight measured at Week 4 in four groups of vagal nerve stimulation with sham stimulation (SS) and stimulation cycles 5-second ON/55-second OFF, 10-second ON/50-second OFF and 20-second ON/40-second OFF.
Fig. 13 is graphs produced by comparing values of left ventricular end-diastolic pressure and left ventricular dP/dt measured at Week 4 in four groups of vagal nerve stimulation with sham stimulation (SS) and stimulation cycles 5-second ON/55-second OFF, 10-second ON/50-second OFF and 20-second ON/40-second OFF.
Fig. 14 is graphs produced by comparing values of left ventricular ejection fraction and quantities of brain natriuretic peptide (BNP) measured at Week 4 in four groups of vagal nerve stimulation with sham stimulation (SS) and stimulation cycles 5-second ON/55-second OFF, 10-second ON/50-second OFF and 20-second ON/40-second OFF.
Fig. 15 is graphs produced by comparing values of biventricular weight and lung weight measured at Week 4 in three groups of vagal nerve stimulation with sham stimulation (SS) and pulse widths 60µsec and 180µsec.
Fig. 16 is graphs produced by comparing values of left ventricular end-diastolic pressure and left ventricular dP/dt measured at Week 4 in three groups of vagal nerve stimulation with sham stimulation (SS) and pulse widths 60µsec and 180µsec.
Fig. 17 is a graph produced by comparing values of left ventricular ejection fraction measured at Week 4 in three groups of vagal nerve stimulation with sham stimulation (SS) and pulse widths 60µsec and 180µsec.
Fig. 18 is photographs showing vagus nerve tissue images at electrode contact portions observed on Day 28 of stimulation.
Fig. 19 is graphs produced by comparing the number of small fibers and the number of large fibers measured for sham stimulation (SS) and voltage values 100%, 50% and 2 5%.
Fig. 20 is an explanatory view showing the detection of the heart rate signal R wave (R1, R2, R3 ...) and the calculation of each R wave interval (Δ1, Δ2 ...).
Fig. 21 (left) is a histogram comparing fluctuations in the R wave interval between the group of non-stimulation and the group of voltage value 5 0%. Fig. 21 (right) is a graph comparing the standard deviation (SD) of the R wave interval between the stimulation of voltage value 50% and the stimulation of voltage value 100% when no-stimulation is set to 1.
Fig. 22 is a block diagram showing the function of the present neurostimulation device in controlling and regulating the quantity of electric or magnetic stimulus.
Fig. 23 is a flow diagram showing one example of controlling and regulating the quantity of stimulus by the control part.

### DETAILED DESCRIPTION OF THE INVENTION

A description of preferable embodiments of the present invention is given below in detail.

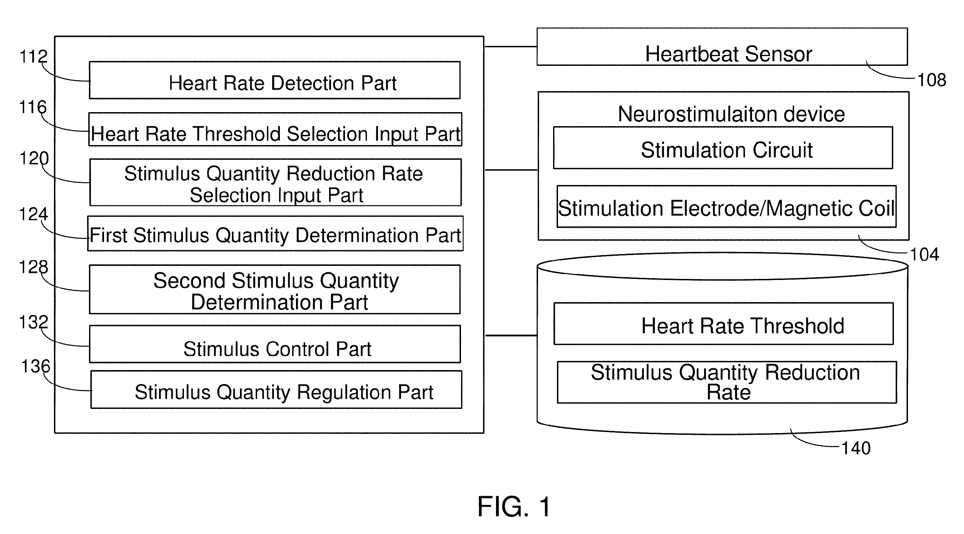

Fig. 1 is a schematic block diagram showing a neurostimulation device according to the present embodiment. A stimulation means may be electric or magnetic and is configured so as to be used for the treatment of circulatory disease. This neurostimulation device comprises a stimulation device 104 configured so as to apply a stimulus to a vagus nerve portion in the cervix or thorax. The neurostimulation device is connected with a heartbeat sensor 108 that can be disposed at a heartbeat measuring portion. The main body of this neurostimulation device is provided with a heartbeat detection part 112, a heart rate threshold selection input part 116, a stimulus reduction rate selection input part 120, a first stimulus quantity determination part 124, a second stimulus quantity determination part 128, a stimulus control part 132 and a stimulus regulation part 136. The device is further provided with a storage part 140, which stores values necessary for stimulus control such as thresholds inputted at the abovementioned heart rate threshold selection input part 116 and stimulus reduction rate values inputted at the stimulus reduction rate selection input part 120. A plurality of these predetermined values may be stored in the storage part 140 in advance so that a user can select one. Alternatively, these values may be inputted manually in a timely manner using an interface connected to the abovementioned heart rate threshold selection input part 116, the abovementioned stimulus reduction rate selection input part 120 and the like to be stored in the abovementioned storage part 140.

In the case of electric stimulation, the neurostimulation device 104 comprises one or more electrodes for applying electric pulses and allows applying an electric stimulus to a cervical or thoracic vagus nerve in an animal through those electrodes. A stimulation circuit may be constituted of, for example, a direct current voltage generation circuit for generating predetermined voltage values, a capacitor to be charged by voltage generated at the direct current voltage generation circuit and a switch disposed between the capacitor and an electrode to be used for disconnecting therebetween.

In the case of magnetic stimulation, the neurostimulation device 104 comprises one or more coils for applying magnetic pulses and allows applying a magnetic stimulus to a cervical or thoracic vagus nerve in an animal through those coils. The neurostimulation device 104 used for magnetic stimulation is configured so as to generate magnetic flux, which fluctuates time-dependently, around a coil, wherein the time-dependently fluctuating magnetic flux generates eddy current at a magnetic stimulation portion of the body, so that effects similar to those of electric stimulation can be produced.

The abovementioned heartbeat sensor 108 measures the heartbeat of a patient and sends the information to the heartbeat detection part 112. This sensor 108 may be an existing one, i.e., an optical sensor, a wireless sensor, or the like may be employed. A device for measuring another life sign value such as blood pressure value, respiratory rate and body temperature may be used in place of the heartbeats.

The present neurostimulation device comprises the heart rate detection part 112 for detecting the heart rate measured by the abovementioned heartbeat sensor 108 and heartbeat-related data such as the R wave interval. Furthermore, for the purpose of controlling the quantity of stimulus as described below, the present neurostimulation device comprises the abovementioned heart rate threshold selection input part 116 for selecting or inputting a heart rate threshold and the abovementioned stimulus reduction rate selection input part 120 for selecting or inputting a reduction rate for reducing and outputting the abovementioned quantity of stimulus. The heart rate threshold may be in the range of 5% to 15%. Moreover, a reduction rate value for setting the quantity of stimulus, which is lowered from a predetermined quantity of stimulus, may be in the rage of 30% to 60%, for example. A plurality of these values may be stored in the abovementioned storage part 140 in advance, so that values can freely be selected. These values may be manually inputted by a user from the input part 116, 120 or the like through the interface function, based on data displayed on a display such as measured values and quantities of stimulus, to be stored in the abovementioned storage part 140.

Furthermore, the present neurostimulation device comprises the first stimulus quantity determination part 124 for determining the first quantity of stimulus obtained from the heart rate detected by the abovementioned heart rate detection part 112 and the abovementioned heart rate threshold, and the second stimulus quantity determination part 128 for determining the second quantity of stimulus, which is lower than the abovementioned first quantity of stimulus at the abovementioned reduction rate. Further provided is the abovementioned stimulus control part 132 for controlling this neurostimulation device in such a manner that a cervical or thoracic vagus nerve portion in a human or an animal can be stimulated using the second quantity of stimulus determined above. Moreover, this control part 132 is configured so as to control stimulus parameters of electric or magnetic stimulus such as frequency, intensity and stimulation cycle as well.

Furthermore, the present neurostimulation device comprises the abovementioned stimulus quantity regulation part 136 in order to regulate the quantity of stimulus based on the abovementioned heart rate before treatment, during treatment and the like. The stimulus quantity regulation part 136 may be connected to the abovementioned stimulus control part 132 or configured so as to be part thereof.

As used herein, the vagus nerve refers to a parasympathetic nerve, which mainly controls the internal organs in the thorax, and is also involved in the regulation of the heart rate, the peristaltic movement of the stomach and intestines, perspiration, speech and the like. Such a vagus nerve starts from the brain stem and reaches the abdomen. In the case of applying an electric stimulus to a vagus nerve portion via an electrode, the vagus nerve portion to which the electric stimulus is to be applied may be exposed and brought into direct contact with the electrode, and then the electric stimulus may be applied. In order to stimulate a vagus nerve, it is also possible to apply an acupuncture stimulus to an acupuncture point or apply an electric stimulus from the vascular endothelium side. On the other hand, as far as a coil for a magnetic stimulus is concerned, its invasiveness is relatively low because a vagus nerve can be stimulated simply by bringing the coil into contact with the skin in the cervix, the thorax or the like.

The electric or magnetic stimulation device according to the present invention for the treatment of circulatory disease is excellent at compactness and operational convenience as described above and, therefore, makes it possible to perform treatment by stimulating a vagus nerve during emergency transportation. If it is possible to perform treatment by stimulating a vagus nerve during emergency transportation, the occurrence of complications can effectively be reduced after ischemic reperfusion.

The electric or magnetic stimulation device according to the present invention for the treatment of circulatory disease can be applied to various circulatory disorders that can be treated by stimulating vagus nerves in the body. By way of example, this device can be applied for the treatment of acute myocardial infarction, angina including unstable angina, cardiac failure, arrhythmia, hypertension and arteriosclerosis and is particularly effective for the treatment of acute myocardial infarction and cardiac failure.

### [Example 1]

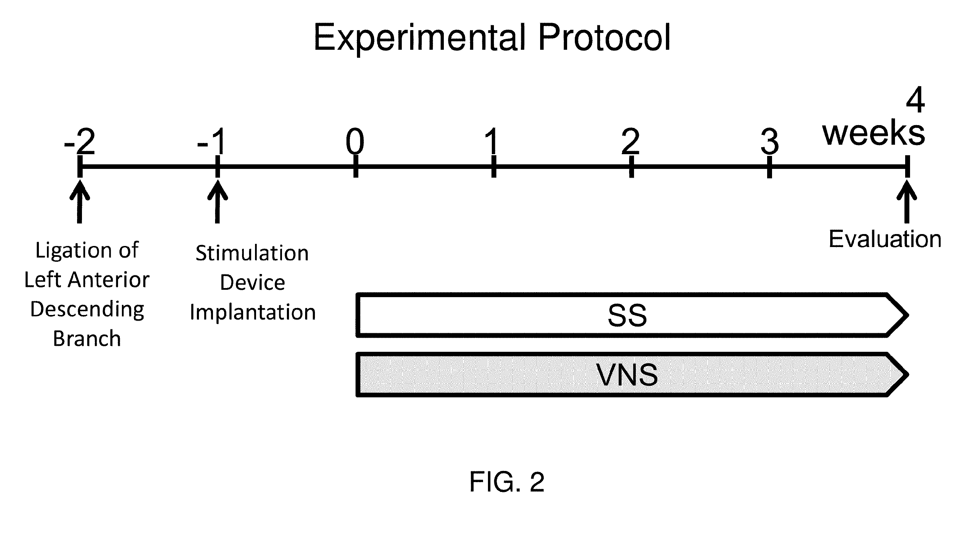

Fig 2 is a view explaining an experimental protocol for the treatment of circulatory disease by applying a stimulation device according to the present embodiment. In the present experiment, myocardial infarction was created by first opening the chest of a male SD (Spraigue-Dowley) rat under anesthesia, ligating the left anterior descending branch of the left coronary artery and then releasing a ligature after ischemia for 30 minutes to perform reperfusion. After one week of the operation, a vagus nerve stimulation device was implanted. More specifically, a right cervical vagus nerve was exposed, and a stimulation device was used that was adjustable to 0-5V in voltage, 0.06-0.18msec in pulse width and 5, 10 and 20Hz in frequency, wherein the stimulation cycle was 5-second ON/55-second OFF, 10-second ON/50-second OFF and 20-second ON/40-second OFF. Also, a telemeter was implanted in the abdomen to monitor the heart rate. One week later, rats were divided at random, and vagal nerve stimulation (VNS) was performed for four weeks. A group with sham stimulation (SS) was also created as a control group, wherein only electrode was attached without applying electric current. Measurement items were evaluated on the 4^{th} week.

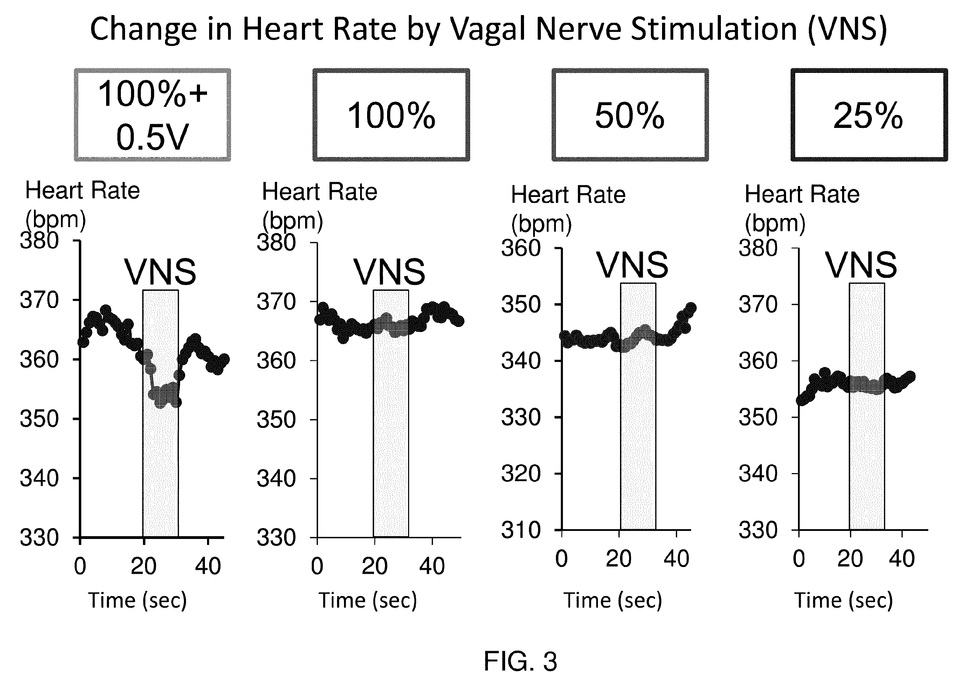

Fig. 3 is graphs showing changes in the heart rate caused by vagal nerve stimulation. A voltage (a first quantity of stimulus) lowered by 0.5V from the lowest voltage at which the heart rate declined by 10% was defined as 100%, and vagal nerve stimulation was applied using four types of voltages, i.e., 100% + 0.5V, 100%, 50% and 25% under the conditions of 20Hz in frequency, 180µsec and 10-second ON/50-second OFF in stimulation cycle. Only in the group applied with a voltage at which the heart rate declined by a maximum of 10% (i.e., 100% + 0.5V), the heart rate significantly declined during stimulation.

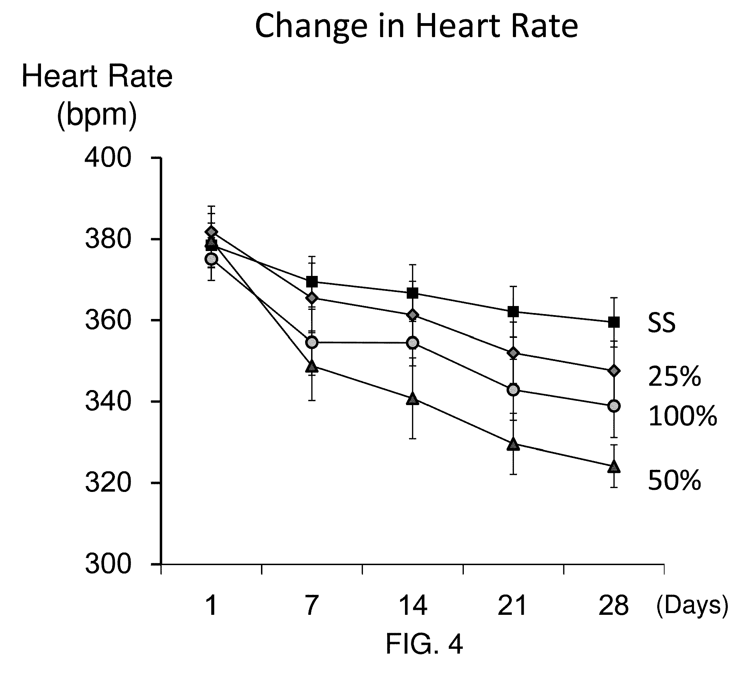

Fig. 4 is a graph produced by plotting and comparing values obtained by measuring changes in the heart rate every week for four weeks in four groups of vagal nerve stimulation with sham stimulation (SS) and voltage values 25 %, 50% and 100%. The stimulation was applied under the conditions of 20Hz in frequency, 180µsec and 10-second ON/50-second OFF in stimulation cycle. In the group of voltage value 50% as the intensity of stimulus, the heart rate significantly declined at the 3^{rd} week and the 4^{th} week, demonstrating that the therapeutic effect could be achieved.

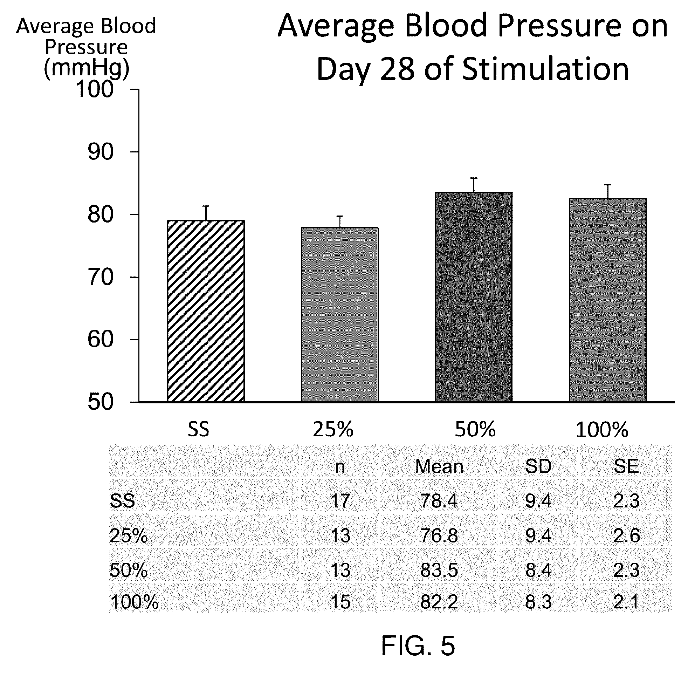

Fig. 5 is a graph produced by comparing values of average blood pressure plotted on Day 28 in four groups of vagal nerve stimulation with sham stimulation (SS) and voltage values 25 %, 50% and 100%. The stimulation was applied under the conditions of 20Hz in frequency, 180µsec and 10-second ON/50-second OFF in stimulation cycle. A table below the graph compares, from among statistic data, the number of rats (n), mean, standard deviation (SD) and standard error (SE) among groups. Among these four groups, there was hardly any significant difference in average blood pressure, nor was any significant decline in blood pressure found even in voltage 50% or 100%.

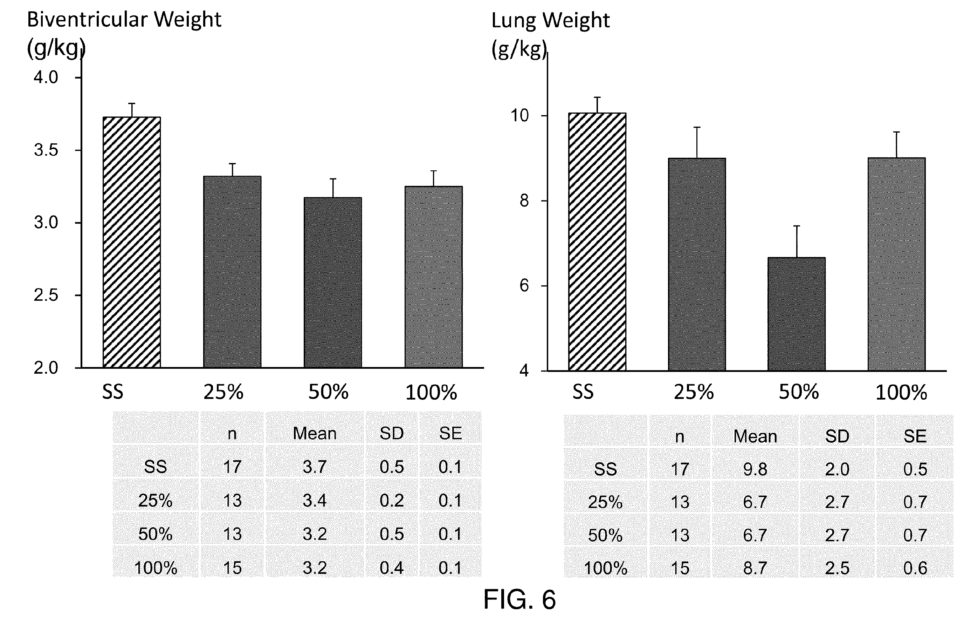

Fig. 6 is graphs produced by comparing values of biventricular weight and lung weight plotted on Day 28 in four groups of vagal nerve stimulation with sham stimulation (SS) and voltage values 25 %, 50% and 100%. The stimulation was applied under the conditions of 20Hz in frequency, 180µsec and 10-second ON/50-second OFF in stimulation cycle. A table below the graph compares, from among statistic data, the number of rats (n), mean, standard deviation (SD) and standard error (SE) among groups. Both the biventricular weight and the lung weight significantly declined in the group applied with the stimulus of voltage value 50%.

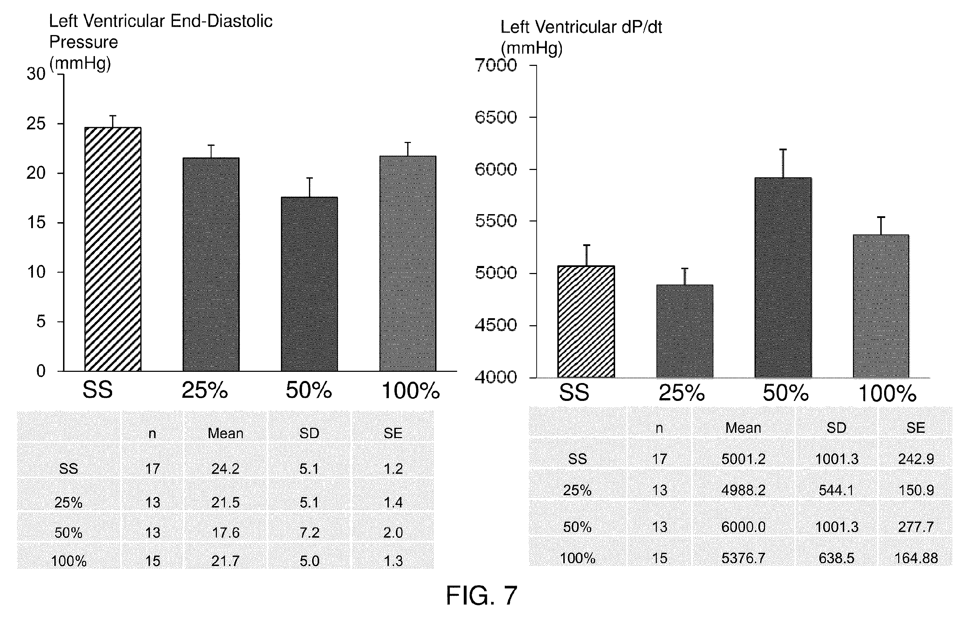

Fig. 7 is graphs produced by comparing values of left ventricular end-diastolic pressure and left ventricular dP/dt (time derivative of left ventricular pressure) plotted on Day 28 in four groups of vagal nerve stimulation with sham stimulation (SS) and voltage values 25 %, 50% and 100%. The stimulation was applied under the conditions of 20Hz in frequency, 180µsec and 10-second ON/50-second OFF in stimulation cycle. Both the left ventricular end-diastolic pressure and the left ventricular dP/dt are indices showing the degree of ventricular contraction. A table below the graph compares, from among statistic data, the number of rats (n), mean, standard deviation (SD) and standard error (SE) among groups. The left ventricular end-diastolic pressure significantly declined and the left ventricular dP/dt significantly increased in the group applied with the stimulus of voltage value 50%.

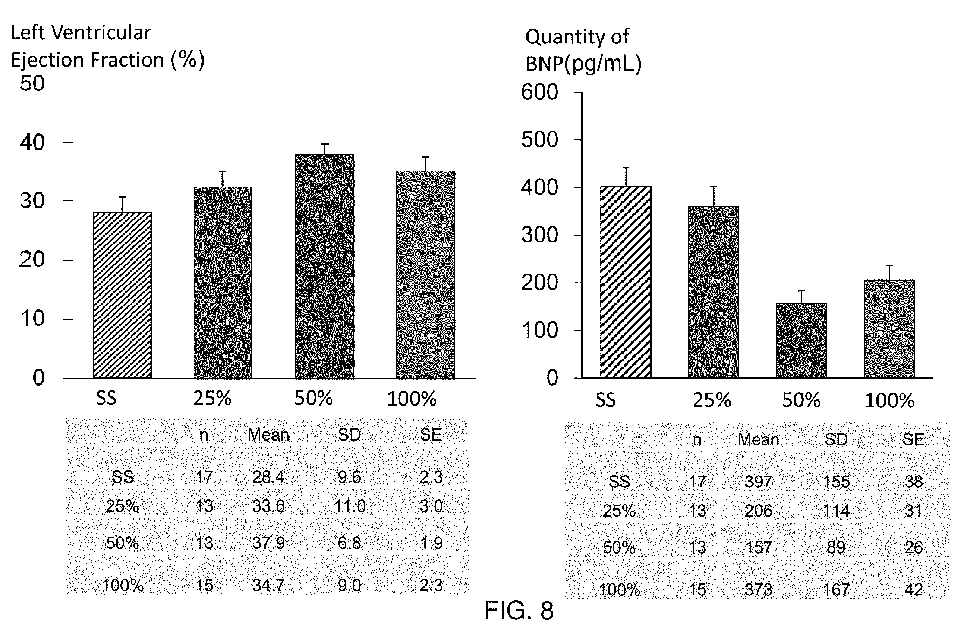

Fig. 8 is graphs produced by comparing values of left ventricular ejection fraction and quantities of brain natriuretic peptide (BNP) plotted on Day 28 in four groups of vagal nerve stimulation with sham stimulation (SS) and voltage values 25 %, 50% and 100%. The stimulation was applied under the conditions of 20Hz in frequency, 180µsec and 10-second ON/50-second OFF in stimulation cycle. A table below the graph compares, from among statistic data, the number of rats (n), mean, standard deviation (SD) and standard error (SE) among groups. The left ventricular ejection fraction significantly increased and the left ventricular dP/dt significantly declined in the group applied with the stimulus of voltage value 50%.

Thus, as a result of examining stimulation voltage, it was determined that voltage value 50% showed the highest anti-remodeling effect, based on the evaluation of biventricular weight, lung weight, left ventricular end-diastolic pressure, left ventricular dP/dt, left ventricular ejection fraction, quantities of brain natriuretic peptide (BNP), etc. after four weeks of stimulation.

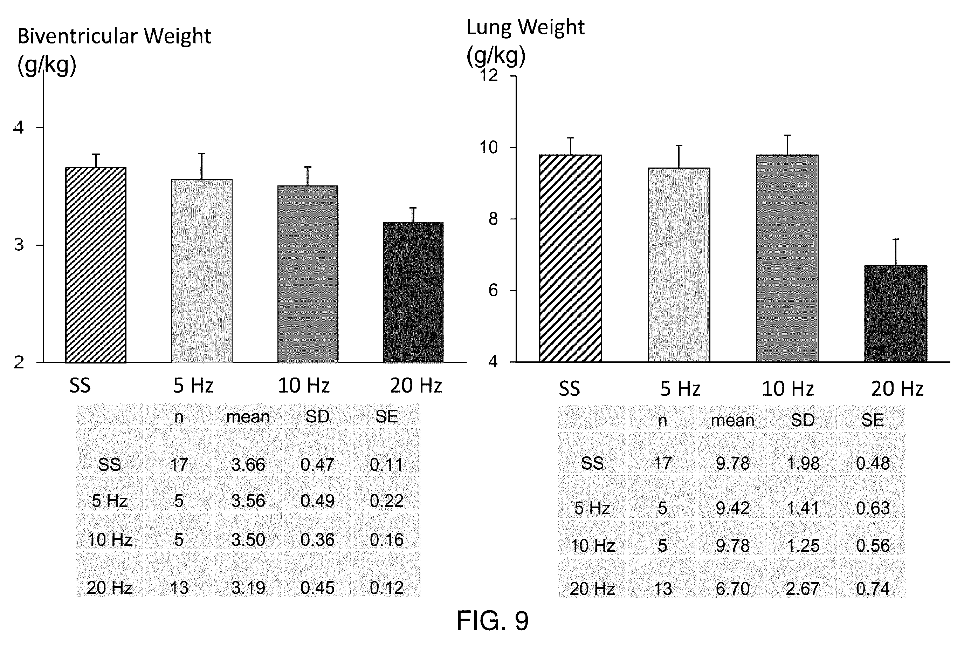

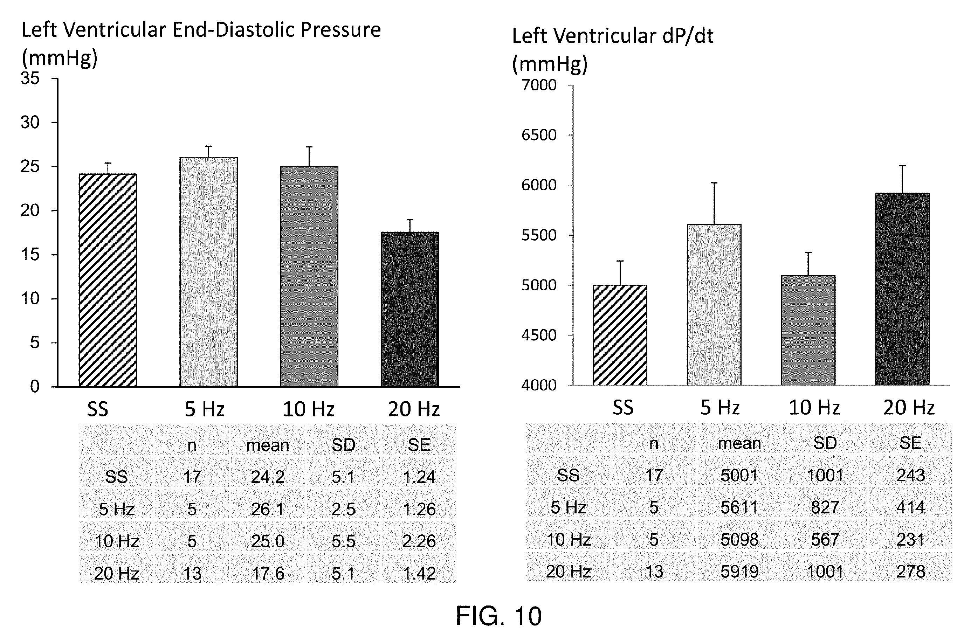

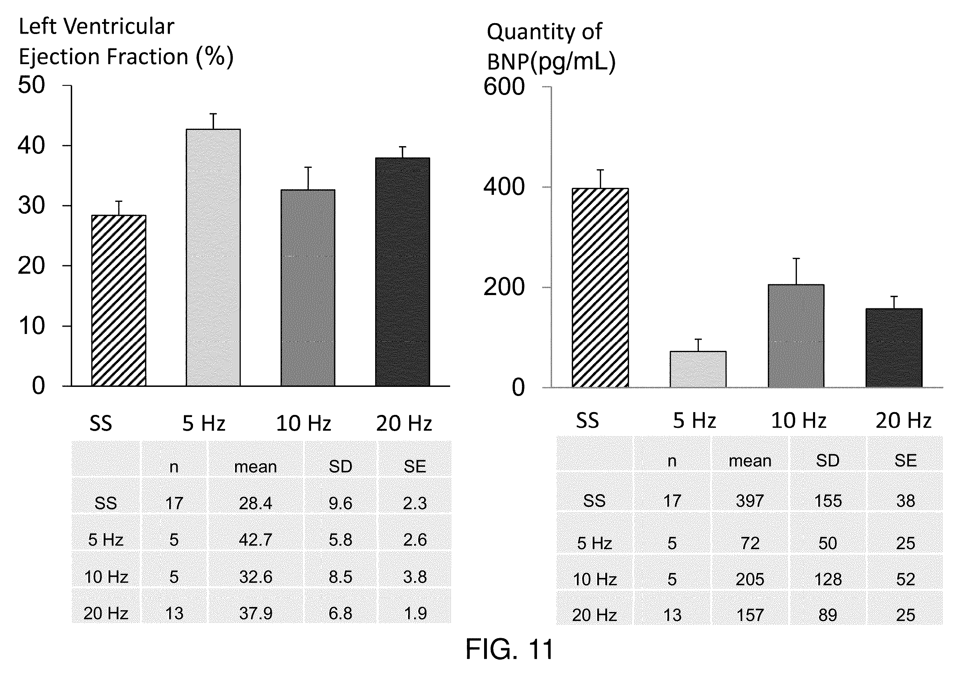

In order to examine stimulation frequency, the voltage value was fixed to 50%, the pulse width to 180µsec and the stimulation cycle to 10-second ON/50-second OFF, and stimulation was applied to the four groups with sham stimulation (SS) and frequencies 5Hz, 10Hz and 20Hz to measure and compare biventricular weight, lung weight, left ventricular end-diastolic pressure, left ventricular dP/dt, left ventricular ejection fraction and quantities of brain natriuretic peptide (BNP) after four weeks of the stimulation. Figs. 9-11 are graphs for comparing the groups applied with the abovementioned four types of stimulation for the abovementioned six types of anti-modeling indices. It was determined that the stimulus of 20Hz in frequency showed the highest anti-remodeling effect.

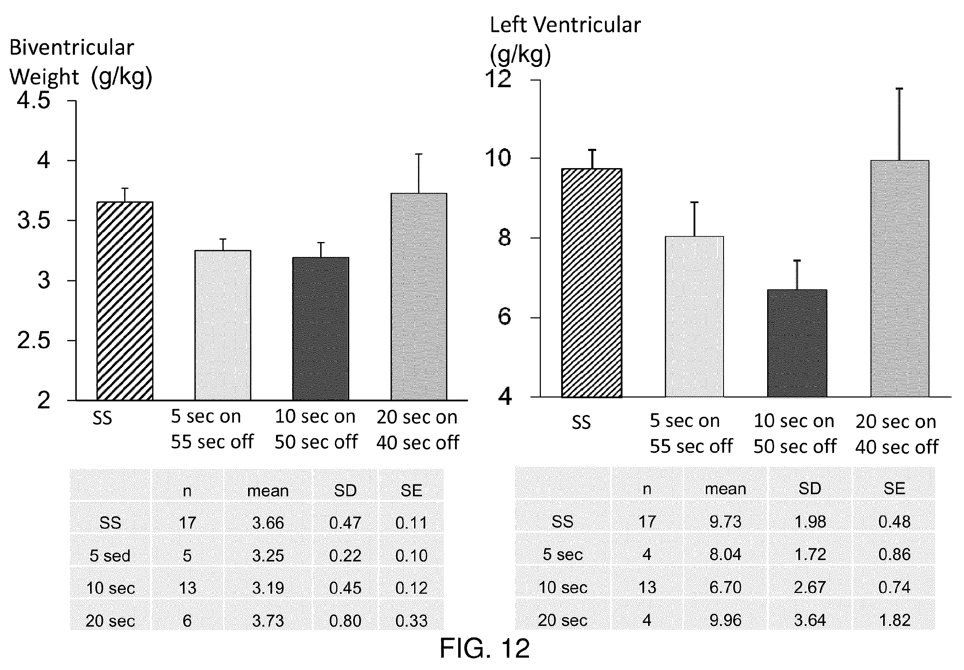

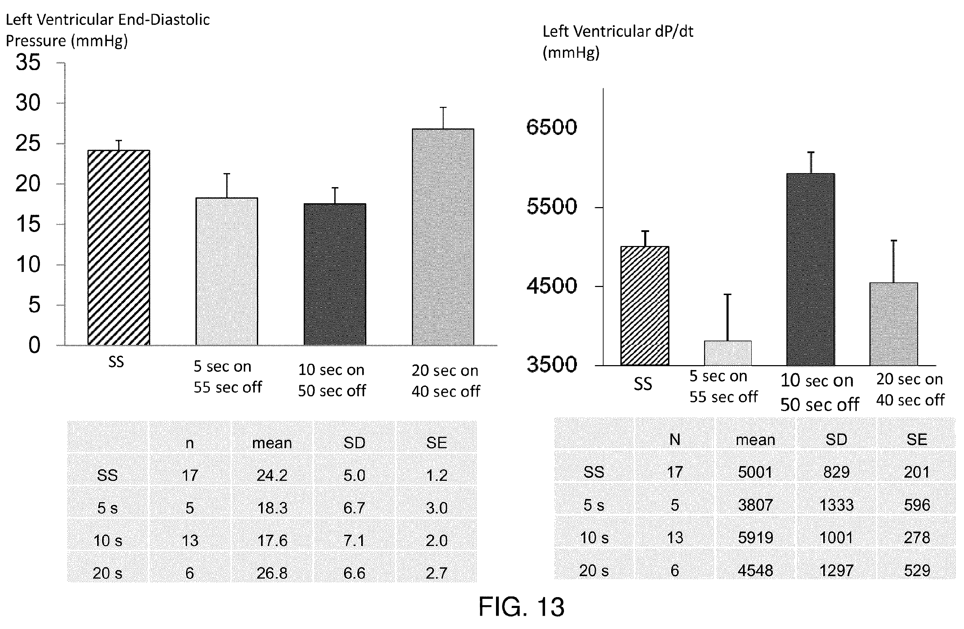

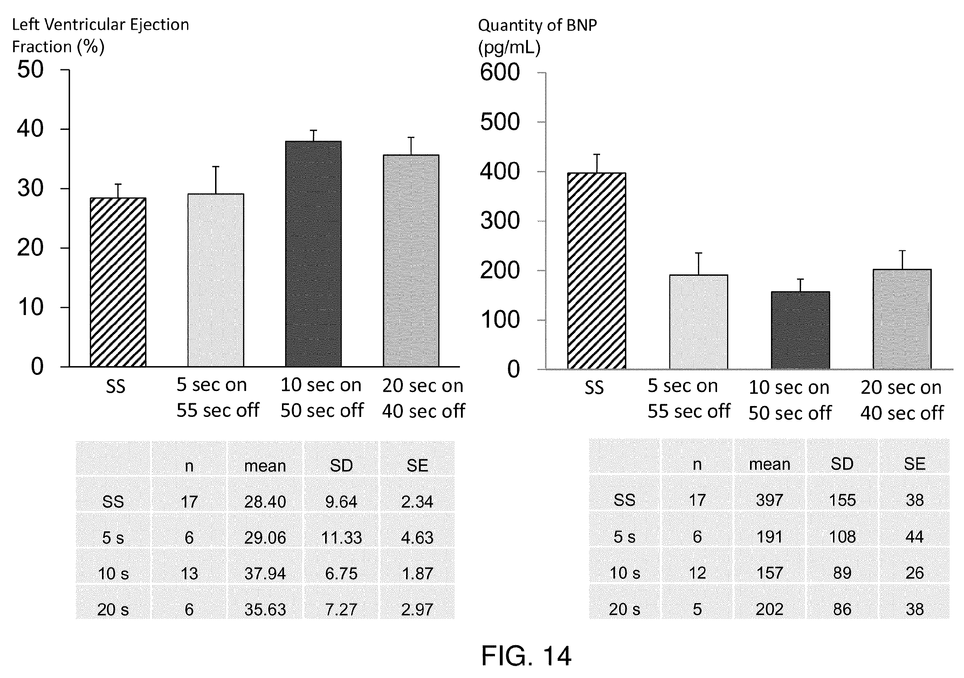

In order to examine stimulation cycle, the voltage value was fixed to 50%, the pulse width to 180µsec and the frequency to 20Hz, and stimulation was applied to the four groups with sham stimulation (SS) and stimulation cycles 5-second ON/55-second OFF, 10-second ON/50-second OFF and 20-second ON/40-second OFF to measure and compare biventricular weight, lung weight, left ventricular end-diastolic pressure, left ventricular dP/dt, left ventricular ejection fraction, quantities of brain natriuretic peptide (BNP) after four weeks of the stimulation. Figs. 12-14 are graphs for comparing the groups applied with the abovementioned four types of stimulation for the abovementioned six types of anti-modeling indices. It was determined that the stimulation cycle 10-second ON/50-second OFF showed the highest anti-remodeling effect.

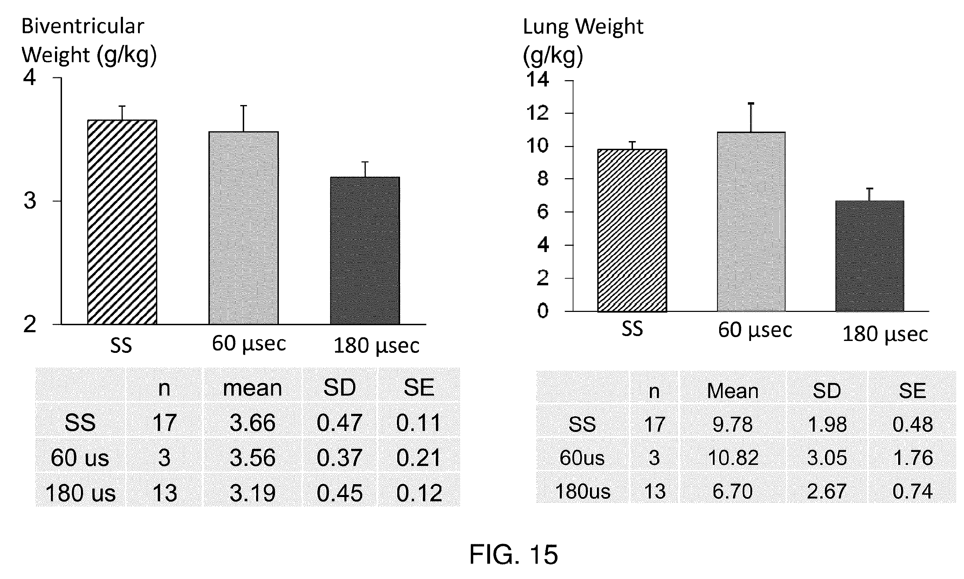

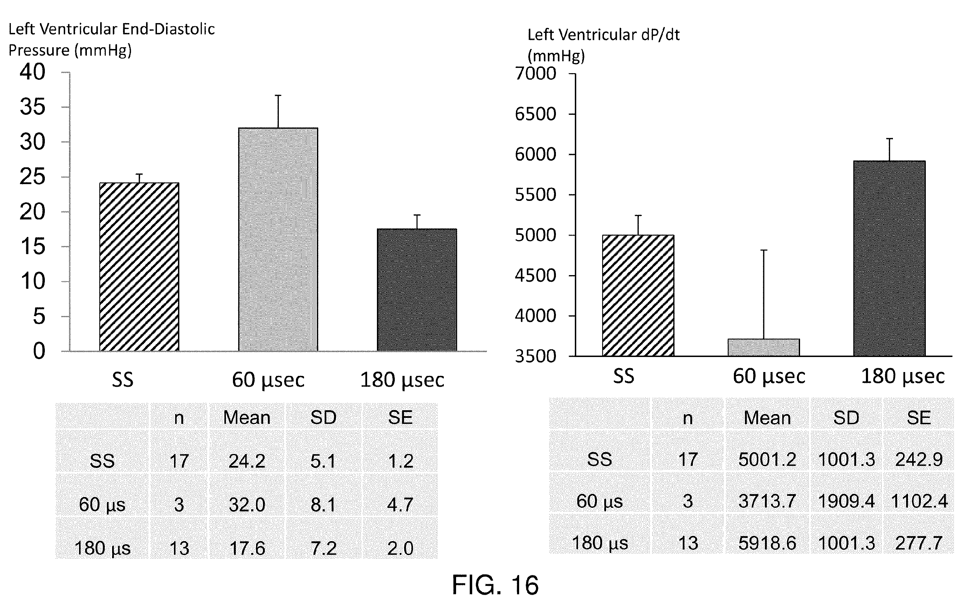

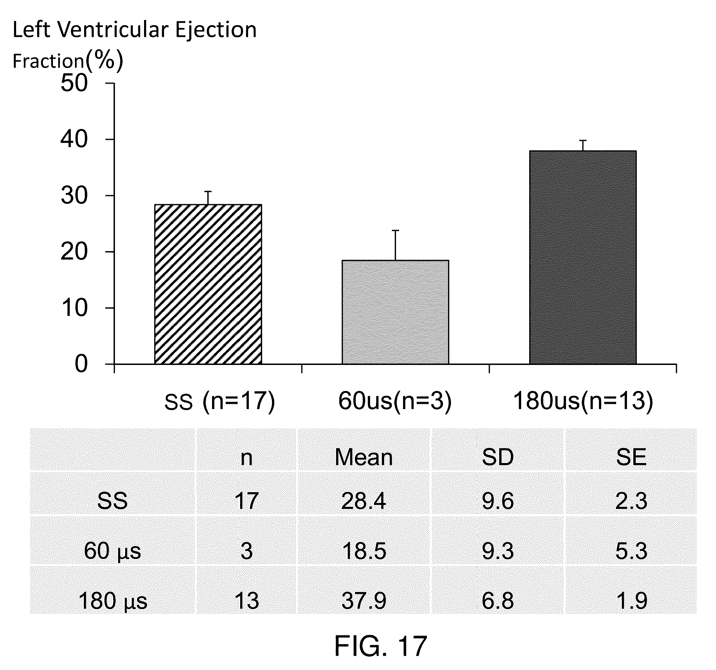

In order to examine pulse width, the voltage value was fixed to 50%, the frequency to 20Hz and the stimulation cycle to 10-second ON/50-second OFF, and stimulation was applied to three groups with sham stimulation (SS) and pulse widths 60µsec and 180µsec to measure and compare biventricular weight, lung weight, left ventricular end-diastolic pressure, left ventricular dP/dt and left ventricular ejection fraction after four weeks of the stimulation. Figs. 15-17 are graphs for comparing the groups applied with the abovementioned three types of stimulation for the abovementioned five types of anti-modeling indices. It was determined that the pulse width 180µsec showed the highest anti-remodeling effect.

Next, in order to examine the influence of stimulation voltage on the nerve tissue, an electrode was wound around a right vagus nerve of a rat, and then a nerve tissue was stained with toluidine blue after applying a stimulus for four weeks to make an observation under an electron microscope. This nerve tissue observation was performed to make a comparison among four groups, i.e., one group with sham stimulation (SS) and the other three groups in which the frequency was fixed to 20Hz, the stimulation cycle to 10-second ON/50-second OFF and the pulse width to180µsec, and stimuli were applied at voltage values 100%, 50% and 25 %.

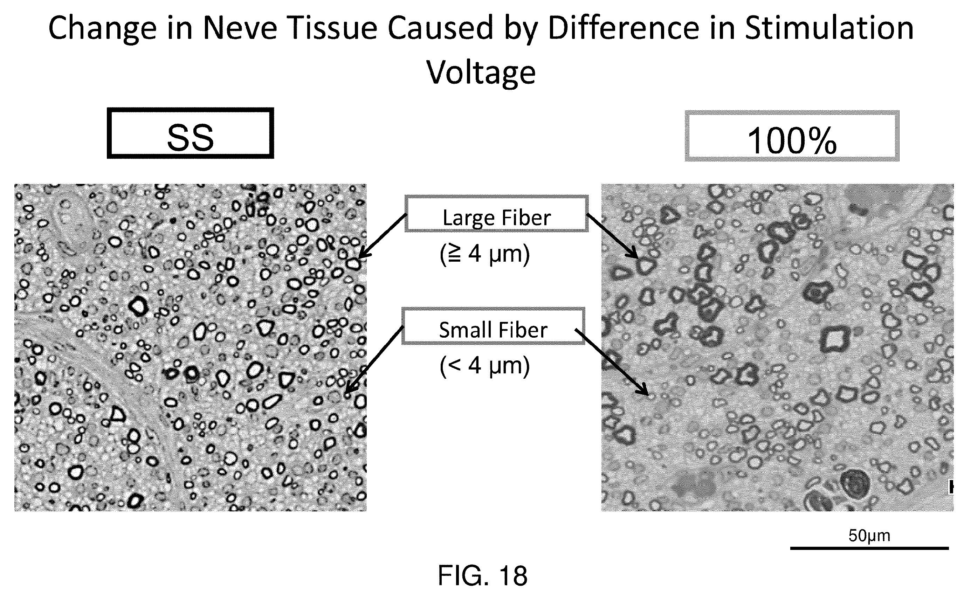

Fig. 18 is photographs showing vagus nerve tissue images at electrode contact portions observed on Day 28 of stimulation. As used herein, fibers equal to or larger than 4µm are referred to as large fibers and fibers less than the abovementioned size as small fibers. It is shown by observation that, as compared with the tissue subjected to sham stimulation, the number of large fibers declined in the case of stimulation at voltage value 100%.

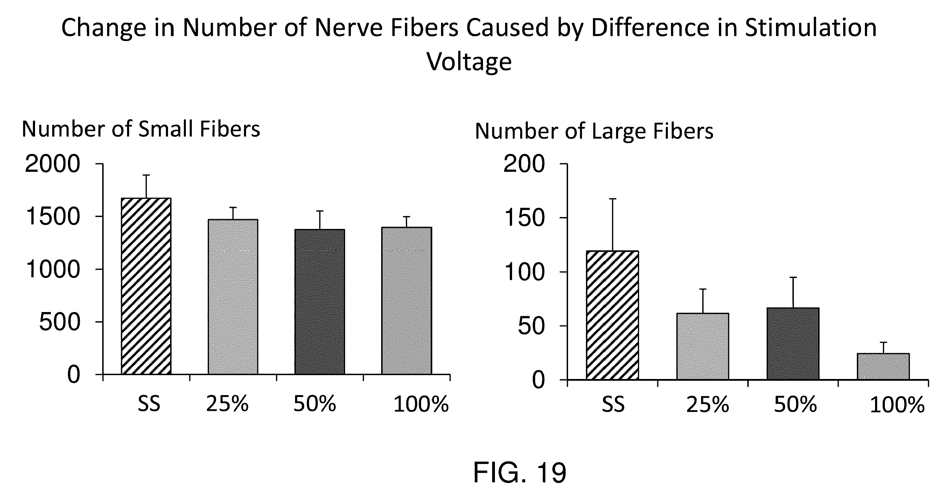

In the abovementioned nerve tissue observation, the number of nerve fibers per 0.1mm² was measured, and the result was examined on the basis of the analysis of variance, or ANOVA, for the abovementioned four groups, i.e., groups subjected to sham stimulation (SS) and the stimulation of voltage values 100%, 50% and 25%. Fig. 19 is graphs produced by comparing the number of small fibers and the number of large fibers measured for sham stimulation (SS) and voltage values 100%, 50% and 2 5% for the abovementioned four groups. In these four groups, no significant difference was found in the number of small fibers of less than 4µm, while there was a significant decline in the number of large fiber of 4µm or more in the group of voltage value 100%. In other words, it was confirmed that damage to the nerve tissue was too significant when the stimulation of voltage value 100% was applied. On the other hand, it was also confirmed that, when the stimulation of voltage value 50% was applied, a reduction in the number of large fiber of 4µm or more was minor and that damage was less as compared with voltage values 100% and 25%.

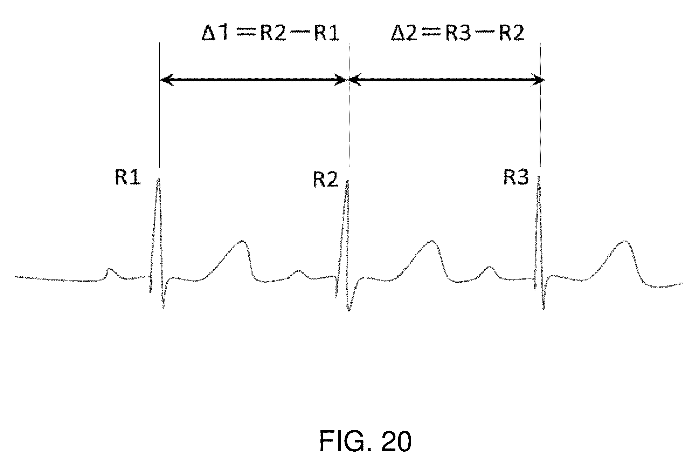

Next, the following examines fluctuations in the R wave interval at the time of vagal nerve stimulation. Fig. 20 is an explanatory view showing the detection of the heart rate signal R wave (R1, R2, R3 ...) and the calculation of each R wave interval (Δ1, Δ2 ...).

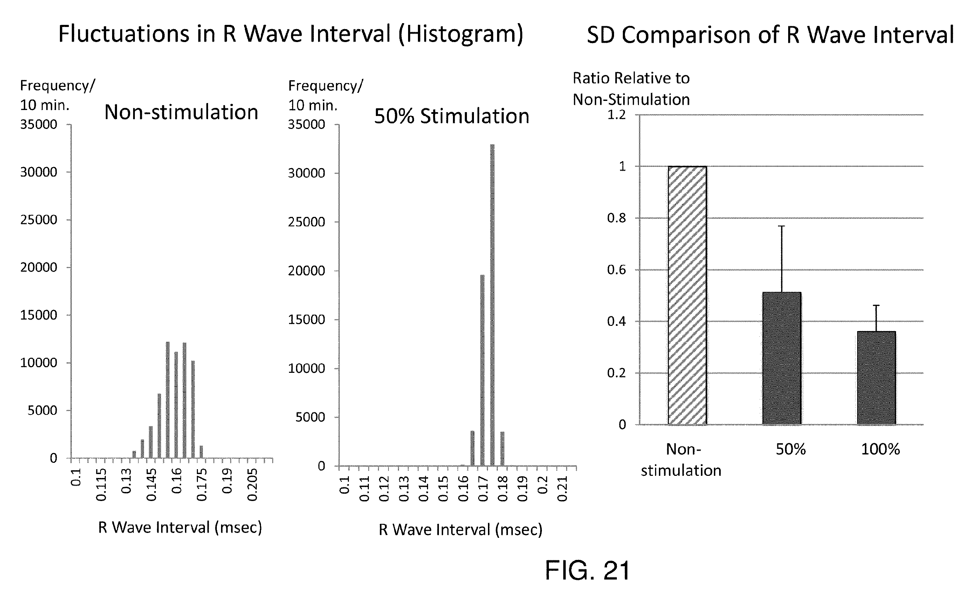

Fig. 21 (left) is a histogram comparing fluctuations in the R wave interval between the group of non-stimulation and the group of voltage value 50%. Fig. 21 (right) is a graph comparing the standard deviation (SD) of the R wave interval between the stimulation of voltage value 50% and the stimulation of voltage value 100% when no-stimulation is set to 1. It was demonstrated that fluctuations in the R wave interval could be suppressed even by the stimulation of voltage value 50%.

Conditions under which a stimulus is applied using an electric or magnetic stimulation device according to the present invention for the treatment of circulatory disease can be selected in consideration of the severity of a patient in an appropriate manner. The quantity of stimulus by voltage, current or the like is set on the basis of the quantity of stimulus that causes a reduction in the heart rate as compared with the heart rate when no stimulus is applied to a vagus nerve. Preferably, the quantity of stimulus that causes the heart rate to decline by about 10% as compared with the heart rate at the time of non-stimulation is found, and then about a half of the abovementioned quantity of stimulus is set to be a quantity of stimulus subjected to be controlled. As a result, it is possible to avoid the conventional therapeutic method in which adverse effects are significant due to an increased quantity of stimulus. It is also possible to avoid the situation where no therapeutic effect can be achieved because the quantity of stimulus is too small.

Additionally, in the therapy using an electric or magnetic stimulation device according to the present invention for the treatment of circulatory disease, it is possible to set a quantity of stimulus that can achieve therapeutic effects by examining fluctuations in the R wave interval as described above, even when a reduction in the heart rate is not obvious while applying a stimulus.

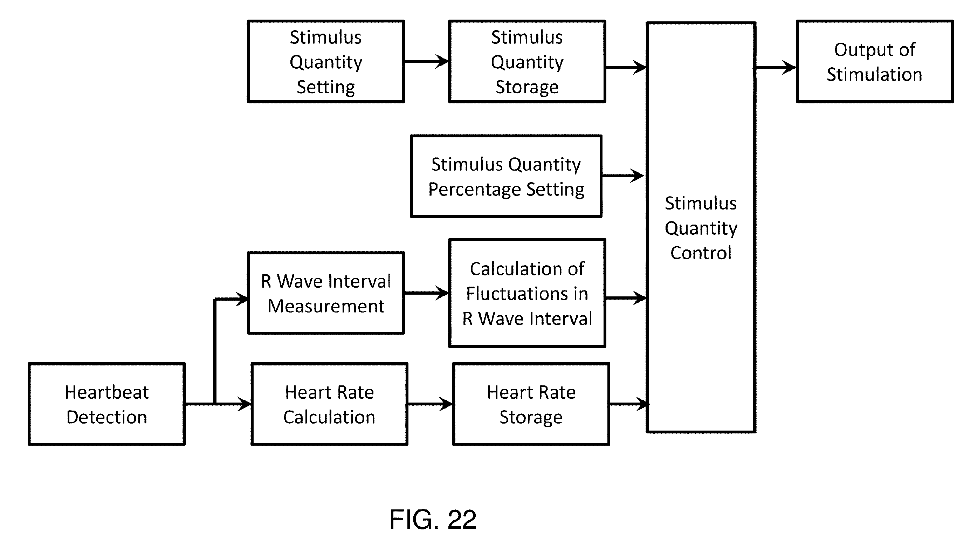

Fig. 22 is a block diagram showing the function of the present neurostimulation device in Fig. 1 at the time of controlling and regulating the quantity of electric or magnetic stimulus. In this case, the abovementioned neurostimulation device is connected with a heartbeat sensor 108 for measuring heartbeats in the body. Alternatively, a device for measuring another biological sign such as blood pressure, respiratory rate and body temperature may be used in place of heartbeats.

First, a heartbeat at the time of non-stimulation is measured by the heartbeat sensor 108, the heartbeat detection part 112 receives this measured value to calculate a heart rate per minute at the time of non-stimulation, and then this value is stored in the storage part 140.

Next, a quantity of stimulus is established. This initial value may manually be inputted using the stimulus quantity reduction rate selection input part 120. Values of the quantity of stimulus are stored in the storage part 140 at predetermined time intervals. The quantity of electric or magnetic stimulus is controlled by the stimulus control part 132 in such a way as to obtain the abovementioned quantity of stimulus, and the neurostimulation device 104 applies a stimulus having the corresponding quantity of stimulus to a vagus nerve portion.

While applying a stimulus, a heartbeat is measured by the heartbeat sensor 108, and, at the heartbeat detection part 112, a heart rate when a stimulus having the abovementioned quantity of stimulus is applied is calculated. When the heart rate at the time of stimulation declines for a predetermined threshold (a declined threshold of biological sign values), the abovementioned stimulus control part 132 establishes the quantity of stimulus as a first quantity of stimulus, displays it on a display or the like and then stores it in the storage part 140. The standard reduction percentage (declined threshold of biological sign values) for determining the first quantity of stimulus may be set to 10%, for example. In order to establish a quantity of stimulus (a second quantity of stimulus) that is lower than the first quantity of stimulus, which causes the heart rate to decline as described above, an appropriate percentage value (e.g., 50%) is retrieved from the storage part 140, and then a quantity of stimulus that is a half of the first quantity of stimulus is established as the second quantity of stimulus and stored in the storage part 140. The stimulus control part 132 performs control in such a manner that an electric or magnetic stimulus having a quantity of stimulus equivalent to the abovementioned first quantity of stimulus multiplied by a selected reduction rate value (e.g., 50%) (i.e., the second quantity of stimulus) can be outputted, and the neurostimulation device 104 applies a stimulus having the corresponding quantity of stimulus to a vagus nerve portion.

At the storage part 140, a plurality of the abovementioned reduction rate values may be established and stored in advance, so that a user can select a value. Alternatively, the configuration may be such that, based on data displayed on a display or the like, a user can manually input a value from the input part 120 via an interface or the like and stores it in the storage part 140.

The abovementioned quantity of stimulus that causes the heart rate to decline is established as the first quantity of stimulus, and it is then confirmed that therapeutic effects can be achieved using the second quantity of stimulus obtained by reducing the abovementioned first quantity of stimulus by a selected percentage value (e.g., 50%). For this purpose, the R wave interval is measured based on heartbeat data detected by the heartbeat detection part 112 to calculate a fluctuation. When the fluctuation is smaller than a predetermined value (e.g., a fluctuation at the time of non-stimulation), it is assumed that therapeutic effects can be achieved by the abovementioned second quantity of stimulus, and the quantity of stimulus is stored in the storage part 140. Furthermore, the stimulus control part 132 performs control in such a manner that an electric or magnetic stimulus having the abovementioned quantity of stimulus can be outputted, and the neurostimulation device 104 applies a stimulus having the corresponding quantity of stimulus to a vagus nerve portion. During the treatment, a user adjusts the quantity of stimulus while detecting heartbeats.

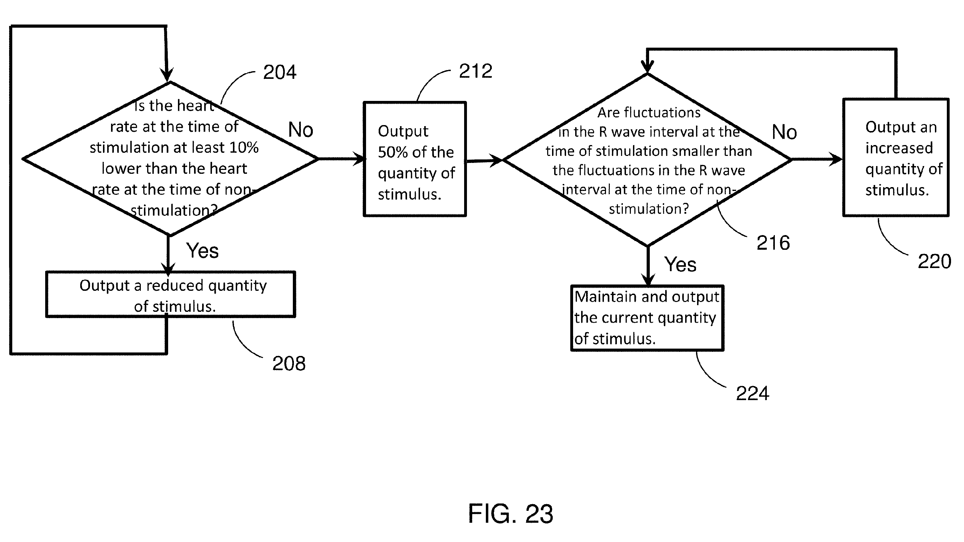

Fig. 23 is a flow diagram showing one example of controlling and regulating the quantity of stimulus by the stimulus control part 132 and the regulation part 136. Heartbeats are continuously measured during the stimulation and non-stimulation, and the heart rate and R wave interval during the non-stimulation are stored in the storage part 140. At S204, it is judged whether or not the heart rate declines at the time of stimulation as compared with non-stimulation. The threshold is set to a 10% decline in the heart rate, for example. This threshold may be stored in the storage part 140 in advance and selected by the heart rate value threshold selection input part 116. Or, it may manually be inputted via an interface or the like from the heart rate value threshold selection input part 116. When the decline is 10% or more, it means that the quantity of stimulus is too high, and therefore the quantity of stimulus must be reduced and then outputted at S208. S204 and S208 are repeated until a decline in heartbeats becomes about 10%. At S212, the quantity of stimulus used when a decline in heartbeats becomes about 10% is set to the first quantity of stimulus and the reduction percentage value to 50%, and therefore a half of the first quantity of stimulus is set to be the second quantity of stimulus and then outputted. Next, at S216, it is judged whether or not a fluctuation in the R wave interval at the time of stimulation is smaller than a predetermined value. The predetermined value may be a fluctuation in the R wave interval at the time of non-stimulation. If the fluctuation at the time of stimulation is large, it means that therapeutic effects have not been achieved, and therefore an increased quantity of stimulus is outputted. However, if the quantity of stimulus becomes equal to or larger than the first quantity of stimulus, it means that the stimulus is too strong and adverse effects or the like might possible occur, and therefore the abovementioned increase must be less than the first quantity of stimulus. Nevertheless, in the abovementioned experimental results shown in Fig. 21, fluctuations in the R wave interval are smaller than those at the time of non-stimulation even when the quantity of stimulus is about a half of the first quantity of stimulus. Accordingly, an increase in the stimulation value at S220 is minute, and fluctuations in the R wave interval become smaller than a predetermined value before the quantity of stimulus increases to a level close to the first quantity of stimulus. If the fluctuations are smaller than a predetermined value, it means that therapeutic effects have been achieved, and therefore the current quantity of stimulus should be maintained and continued to be outputted at S224.

In the abovementioned example of controlling a stimulus, the heart rate is measured, but it may instead be the measurement of another biological sign value such as blood pressure, perspiration rate and body temperature. Moreover, while the threshold of the quantity of stimulus that causes heartbeats to decline is set to a quantity of stimulus when the heart rate declines by 10% from the heart rate at the time of non-stimulation, it may be 5-15% instead. Moreover, while the reduction percentage from the threshold of the quantity of stimulus is set to 50%, it may be 30- 60% instead. In this case, it is preferred that one value in 5-15% and one value in 30-60% can be selected by a pull-down system on a user interface.

In the present invention, the period during which a stimulus is applied to a nerve can appropriately be selected depending on the severity and the like of a patient. Intermittent stimulation may be repeated for 0.1-10 hours or more and preferably for 0.5-10 hours, wherein the stimulation cycle is such that the abovementioned stimulus is applied for 10 seconds per minute and then no stimulus is applied for the remaining 50 seconds. The stimulus may be electric or magnetic. It may be applied intermittently at predetermined time intervals or applied continuously for a predetermined period. Or, after applying a stimulus for a predetermined period, a stimulus may be applied again when some symptoms such as arrhythmia occur. In the case of electric stimulation, the quantity of stimulus may be voltage or current.

Furthermore, the method of the present invention for the treatment of circulatory disease can be combined with reperfusion therapy. In the case of combining the present therapeutic method with reperfusion therapy, a stimulus may be applied prior to reperfusion therapy, or at the same time as reperfusion therapy, or subsequent to reperfusion therapy, but it is preferred that a stimulus be applied prior to reperfusion therapy in the present invention.

In place of the abovementioned electric stimulation, magnetic stimulation may be applied to a vagus nerve portion. In the case of magnetic stimulation, the neurostimulation device 104 in Fig. 1 has one or more coils for applying magnetic pulses, and the configuration of the device is such that magnetic stimulation is applied to a cervical or thoracic vagus nerve in an animal using the abovementioned coils. The neurostimulation device 104 is configured so as to generate magnetic flux, which fluctuates time-dependently, around a coil, wherein the time-dependently fluctuating magnetic flux generates eddy current at a magnetic stimulation portion of the body. It is desirable to configure the device in such a manner as to generate an eddy current density of 15mA/cm² or less to the biological tissue to which a stimulus is applied. During the magnetic pulse stimulation, the heart rate declines so that the effect equivalent to that of the abovementioned electric stimulation can be achieved. It has been demonstrated that the heart rate declines by applying continuous magnetic pulses having a magnetic flux density (B) of 2 tesla or so. As in the case of the abovementioned electric stimulation, the intensity of magnetic flux density of magnetic pulses is set to a first intensity when the heart rate declines by 10% from the heart rate at the time of non-stimulation, and about a half of the first intensity is outputted. As shown in Fig. 23, when fluctuations in the R wave interval are large, the intensity of magnetic flux density is controlled in such a manner that the magnetic flux density is increased less than the first intensity. At a time when fluctuations in the R wave interval has become smaller than a predetermined value at a certain quantity of stimulus, the quantity of stimulus is maintained and continued to be outputted.

It goes without saying that the present invention can be modified in various manners without being limited by the abovementioned embodiment as far as those modifications do not depart from the scope of the present invention.

## Claims

**1.** A neurostimulation device configured so as to stimulate a cervical or thoracic vagus nerve portion in a human or an animal with a controlled quantity of stimulus by applying electric signals to an electrode or a magnetic coil attached to the cervical or thoracic portion in the human or animal, the neurostimulation device comprising:
a biological sign value detection part for detecting biological sign values of the human or animal;
a threshold storage part for storing thresholds of the biological sign values;
a stimulus reduction quantity/reduction rate storage part for storing the reduction quantity/reduction rate of the quantity of stimulus;
a first stimulus quantity determination part for determining a first quantity of stimulus obtained from a threshold stored in the threshold storage part, based on a biological sign value detected by the biological sign value detection part;
a second stimulus quantity determination part for determining a second quantity of stimulus obtained by reducing the first quantity of stimulus by a reduction quantity/reduction rate stored in the stimulus reduction quantity/reduction rate storage part; and
a control part for controlling the neurostimulation device in such a manner as to stimulate the cervical or thoracic vagus nerve portion in the human or animal by the second quantity of stimulus thus determined.

**3.** The neurostimulation device according to Claim 1, wherein the control part comprises a stimulus quantity regulation part that regulates the first quantity of stimulus based on the biological sign value while detecting the biological sign value.

**4.** The neurostimulation device according to Claim 1, further comprising a reduction quantity/reduction rate input interface for the quantity of stimulus, so that a user can input a reduction quantity/reduction rate of the quantity of stimulus.

**5.** The neurostimulation device according to Claim 1, wherein the reduction quantity/reduction rate of the quantity of stimulus is about 50%.

**6.** The neurostimulation device according to Claim 1, further comprising a biological sign value threshold input interface, so that a user can input a threshold of the biological sign value.

**7.** The neurostimulation device according to Claim 1, wherein the threshold is about 10%.

**8.** The neurostimulation device according to Claim 1, wherein the biological sign value to be detected by the biological sign value detection part is a heart rate.

**9.** The neurostimulation device according to Claim 8, wherein: the biological sign value detection part detects an R wave interval of heartbeats in addition to the heart rate; and the control part calculates a fluctuation in the R wave interval, determines whether or not the fluctuation in the R wave interval is smaller than a predetermined fluctuation and, if it is not smaller, regulates the second quantity of stimulus until the R wave interval becomes smaller than the predetermined fluctuation.

**10.** The neurostimulation device according to Claim 9, wherein the predetermined fluctuation is a fluctuation of the R wave interval at a time when no stimulation is applied.

**11.** The neurostimulation device according to Claim 1, wherein the control part controls the neurostimulation device in such a manner that a stimulus can be applied intermittently or continuously based on the second quantity of stimulus.

**12.** The neurostimulation device according to Claim 11, wherein the control part applies a stimulus at a stimulation cycle in which the stimulus is applied continuously for about 10 seconds per minute and no stimulus is applied for the remaining 50 seconds or so.

**13.** The neurostimulation device according to Claim 1, wherein the control part controls the neurostimulation device in such a manner that a stimulus can be applied before reperfusion, after reperfusion or in combination with reperfusion.

**14.** The neurostimulation device according to Claim 1, wherein the control part controls the neurostimulation device in such a manner as to apply a stimulus at a frequency of about 20Hz.

**15.** The neurostimulation device according to Claim 1, wherein the stimulus includes electric pulses and the quantity of stimulus is a voltage.

**16.** The neurostimulation device according to Claim 1, wherein the stimulus includes electric pulses and the quantity of stimulus is an electric current.

**17.** The neurostimulation device according to Claim 1, wherein the stimulus includes magnetic pulses and the quantity of stimulus is a magnetic flux density.

**18.** The neurostimulation device according to Claim 17, wherein the control part regulates the magnetic flux density in such a manner that electric current generated in the biological tissue by the magnetic flux density is 15mA/cm² or less.

**19.** A nerve stimulation method for stimulating a cervical or thoracic vagus nerve portion in a human or an animal, the method comprising:
a biological sign value detection step of detecting a biological sign value of the human or animal;
a threshold storage step of storing a threshold of biological sign values;
a stimulus reduction quantity/reduction rate storage step of storing the reduction quantity/reduction rate of the quantity of stimulus;
a first stimulus quantity determination step of determining a first quantity of stimulus obtained from a threshold stored in the threshold storage step, based on a biological sign value detected in the biological sign value detection step;
a second stimulus quantity determination step of determining a second quantity of stimulus obtained by reducing the first quantity of stimulus by a reduction quantity/reduction rate stored in the stimulus reduction quantity/reduction rate storage step; and
a control step of controlling the quantity of stimulus in such a manner as to stimulate the cervical or thoracic vagus nerve portion in the human or animal by the second quantity of stimulus thus determined.

**20.** The nerve stimulation method according to Claim 19, wherein the biological sign value to be detected in the biological sign value detection step is a heart rate.

**21.** The nerve stimulation method according to Claim 20, wherein: the biological sign value detection step comprises a step of detecting an R wave interval of heartbeats in addition to the heart rate; and the control step comprises steps of calculating a fluctuation in the R wave interval, determining whether or not the fluctuation in the R wave interval is smaller than a predetermined fluctuation and, if it is not smaller, regulating the second quantity of stimulus until the R wave interval becomes smaller than the predetermined fluctuation.

**22.** The nerve stimulation method according to Claim 19, wherein the reduction quantity/reduction rate of the quantity of stimulus is about 50%.

**23.** The nerve stimulation method according to Claim 19, wherein the threshold is about 10%.

**24.** The nerve stimulation method according to Claim 21, wherein the predetermined fluctuation is a fluctuation of the R wave interval at a time when no stimulation is applied.

**25.** The nerve stimulation method according to Claim 19, wherein the control step is a step of controlling the quantity of stimulus in such a manner that a stimulus can be applied before reperfusion, after reperfusion or in combination with reperfusion.

**26.** The nerve stimulation method according to Claim 19, wherein the stimulus includes electric pulses and the quantity of stimulus is a voltage.

**27.** The nerve stimulation method according to Claim 19, wherein the stimulus includes electric pulses and the quantity of stimulus is an electric current.

**28.** The nerve stimulation method according to Claim 19, wherein the stimulus includes magnetic pulses and the quantity of stimulus is a magnetic flux density.
